# EUROPÄISCHE PATENTSCHRIFT

(11) **EP 1 440 649 B1**
(45) Veröffentlichungstag und Bekanntmachung des Hinweises auf die Patenterteilung: **15.08.2007**
(21) Anmeldenummer: 04000389.9
(22) Anmeldetag: 10.01.2004
(51) Int. Cl.: A47K 3/30, F21V 33/00, F21Y 101/02

(54) **Lichtabgabeeinrichtung**
Light emitting device
Dispositif d'émission de lumière

(30) Priorität: 17.01.2003 DE 10302343
(43) Veröffentlichungstag der Anmeldung: 28.07.2004
(73) Patentinhaber: Hansgrohe AG, 77761 Schiltach (DE)
(72) Erfinder: Gross, Jürgen, 77799 Ortenberg (DE)
(74) Vertreter: Schöndorf, Jürgen

(56) Entgegenhaltungen:
- DE-A1- 10 312 866
- DE-A1- 19 901 669
- DE-U1- 20 101 460
- DE-U1- 29 712 283
- US-A- 4 947 291

## Beschreibung

Die Erfindung geht aus von einer Duschkabine mit einer Einrichtung zur Erzeugung von bestimmten Lichterscheinungen.

Es ist bekannt, in Duschkabinen Licht bestimmter Farben oder Farbmischungen zu erzeugen, wobei sich die Farbmischungen und/oder Helligkeiten mehr oder weniger langsam ändern lassen. Diese Änderung kann automatisch geschehen.

Im einfachsten Fall werden hierzu Farbfilter verwendet, die mechanisch über einer Lichtquelle bewegt werden.

Ebenfalls bekannt ist die Verwendung von LED's, die durch einen Glasdiffuser abgeschirmt sind, und die Lichtmischung erfolgt über die Ansteuerung der LED's, die nur einfarbiges Licht abgeben können.

Bei der Verwendung von LED's besteht die Gefahr, dass ein Benutzer der Einrichtung die einzelnen LED's erkennen kann, die dann als farbige Punkte erscheinen. Damit entsteht dann aber keine Mischfarbe.

Es ist bereits ein Brausekopf mit darin angeordneten Glühbirnen bekannt (US 6439472), die zum Teil in die Abstrahlrichtung der Brause abstrahlen. Sie sollen das Brausewasser zu dekorativen Zwecken beleuchten.

Weiterhin bekannt ist eine Brause mit einem Brausekopf, durch den das Wasser fließt und dabei über eine Turbine einen Generator antreibt. Dieser liefert Strom zur Versorgung von LEDs, die hinter der Strahlscheibe angeordnet sind und dadurch die austretenden Wasserstrahlen direkt und indirekt beleuchten (DE 20101460).

Der Erfindung liegt die Aufgabe zugrunde, eine Möglichkeit zur Abgabe von Licht- und Farbsignalen zu schaffen, die bei einfachem Aufbau sich für die Unterbringung in einer Duschkabine besonders gut eignet.

Zur Lösung dieser Aufgabe schlägt die Erfindung eine Lichtabgabeeinrichtung mit den in Anspruch 1 genannten Merkmalen vor. Weiterbildungen der Erfindung sind Gegenstand von Unteransprüchen.

Die Lichtquellen, die jeweils einfarbiges Licht erzeugen, werden mit geringem seitlichen Abstand angeordnet. Das Farbmuster, also beispielsweise die Farben rot, grün und blau, werden in einem sich regelmäßig wiederholenden Muster erzeugt. Dadurch wird es möglich, tatsächlich eine Mischung der Grundfarben so durchzuführen, dass der Benutzer eine Mischfarbe sieht. Je nach Ansteuerung kann damit beispielsweise auch weisses Licht erzeugt werden, oder Licht beliebiger Farben. Dies hängt von der Dauer und Intensität der Ansteuerung der einzelnen Grundfarben ab.

Am sinnvollsten ist es, wenn von den Lichtquellen jeder Farbe die gleiche Anzahl vorhanden ist, wobei dann vorausgesetzt ist, dass alle Lichtquellen dann die gleiche Charakteristik aufweisen. Es ist aber natürlich auch möglich, die Zahl der Lichtquellen einer bestimmten Farbe zu verringern oder zu vergrössern, wenn diese Lichtquellen eine andere Charakteristik aufweisen oder angesteuert werden.

In Weiterbildung der Erfindung kann vorgesehen sein, dass die Lichtquellen flächig angeordnet sind, vorzugsweise in einer ebenen Fläche. Dies ist aus herstellungstechnischen Gründen bevorzugt.

In Weiterbildung der Erfindung kann vorgesehen sein, dass die Lichtabgabeeinrichtung eine Hauptlichtabgaberichtung aufweist, die also beispielsweise quer zu einer Austrittsfläche verläuft.
Die Lichtquellen zur Abgabe des jeweils einfarbigen Lichts werden vorzugsweise derart angeordnet, dass ein Benutzer die Lichtquelle nicht direkt sehen kann. Zum Umlenken der ausgehenden Lichtstrahlen kann ein Reflektor verwendet werden.

Insbesondere kann vorgesehen sein, dass die Lichtabgabeeinrichtung eine Strahlaustrittsscheibe aufweist, die insbesondere mindestens angenähert eben ausgebildet ist. Diese Strahlaustrittsscheibe kann vorzugsweise als Diffuser ausgebildet sein, also aus einem durchscheinenden aber nicht vollständig durchsichtigen Material. Dies dient in Kombination mit der flächigen Anordnung der Lichtquellen mit geringem Abstand zur weiteren Verbesserung des Erscheinungsbilds des abgegebenen Lichts, so dass dann mit Sicherheit erreicht wird, dass keine einzelnen Lichtpunkte mehr sichtbar sind.

Die Lichtquellen können beispielsweise in mindestens einer Reihe angeordnet werden. In dieser Reihe wiederholt sich die Reihenfolge der Farben immer wieder, also beispielsweise rot, grün und blau.

In nochmaliger Weiterbildung kann vorgesehen sein, dass die Lichtquellen in mehreren parallelen Reihen angeordnet sind, wobei dann vorzugsweise das Farbmuster mindestens zweier benachbarter Reihen um ein Raster versetzt ist. Die Lichtquellen sind dann in Reihen und Spalten angeordnet, so dass sich durch die Versetzung des Farbmusters zwischen benachbarten Reihen auch eine Wiederholung des Farbmusters in den Spalten ergibt.

Um die Lichtabgabeeinrichtung möglichst flach ausbilden zu können und gleichzeitig die gewünschten Farbmischungen wirkungsvoll zu erreichen, kann erfindungsgemäss vorgesehen sein, dass die Lichtquellen an den Umfangsflächen eines Zylinders und/oder Quaders angeordnet sind. Hierbei handelt es sich mit Vorteil um einen flachen Zylinder bzw. flachen Quader, so dass die Abstrahlrichtung der Lichtquellen praktisch radial nach aussen verläuft, von wo aus die gegebenenfalls über einen Reflektor in eine Richtung durch den Diffuser umgelenkt werden können. Gegebenenfalls kann statt eines Zylinders oder Quaders auch ein flacher Kegelstumpf oder eine flache Pyramide verwendet werden, was durch entsprechende Ausgestaltung eines Reflektors zu der gleichen Wirkung führen kann.

Erfindungsgemäss kann in der Mitte der Lichtabgabeeinrichtung eine Sanitärbrause angeordnet sein, bei der es sich um eine flach ausgebildete Seiten- und/oder Kopfbrause handeln kann. Es entsteht dadurch eine flache Baueinheit mit einer Brause und einer Brause umgebenden Abgabeeinrichtung. Diese flache Baueinheit kann an der Oberseite oder auch an den Seitenwänden einer Duschkabine angeordnet werden. Der Benutzer kann dann sowohl das Brausewasser als auch die Lichterscheinungen auf sich einwirken lassen, und zwar gleichzeitig. Es kann dadurch eine Chromotherapie beim täglichen Duschen durchgeführt werden. Durch den flachen Aufbau und die flächige Anordnung vieler Lichtquellen kann auch eine hohe Lichtintensität erreicht werden.

Es kann in Weiterbildung der Erfindung vorgesehen sein, dass die Lichtabgabeeinrichtung zusätzliche weisses Licht abgebende Lampen aufweist, beispielsweise Niederspannungslampen. Diese können die Lichtintensität verstärken.

Weitere Merkmale, Einzelheiten und Vorzüge der Erfindung ergeben sich aus den Patentansprüchen und der Zusammenfassung, deren beider Wortlaut durch Bezugnahme zum Inhalt der Beschreibung gemacht wird, der folgenden Beschreibung einer bevorzugten Ausführungsform der Erfindung sowie anhand der Zeichnung. Hierbei zeigen:
- Fig. 1: in stark vereinfachter Darstellung eine Duschkabine mit drei Lichtabgabeeinrichtungen;
- Fig. 2: in größerem Maßstab einen Schnitt durch eine Lichtabgabeeinrichtung nach der Erfindung;
- Fig. 3: eine schematische Draufsicht auf einen Teil der Lichtabgabeeinrichtung;
- Fig. 4: eine Frontansicht eines Elements der Lichtabgabeeinrichtung.

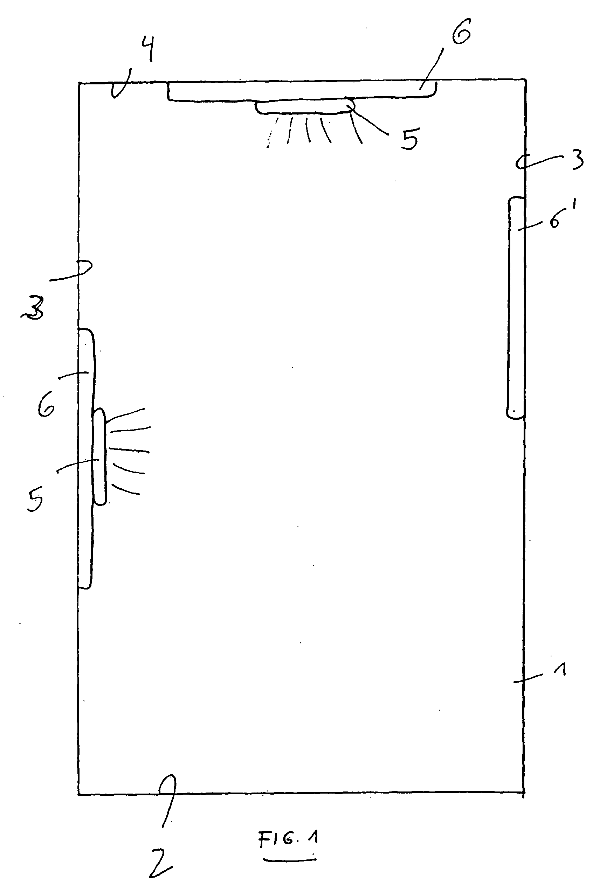

Zunächst zu Fig. 1. Dort ist schematisch eine Duschkabine 1 dargestellt, die aus einem Boden 2, zwei Seitenwänden 3 und einer Oberseite 4 zusammengesetzt ist. An der Oberseite 4 ist, nach unten gerichtet, eine Kopfbrause 5 angebracht. Die nach unten das Brausewasser abgebende Kopfbrause 5 ist mittig in einer Baueinheit 6 angeordnet, die zur Abgabe von Licht ausgebildet ist und im weiteren noch beschrieben werden wird.

Die gleiche Baueinheit 6 mit einer Brause 5 ist an der linken Seitenwand 3 angeordnet, etwa auf der Hälfte der Höhe.

Hierbei handelt es sich also um eine Seitenbrause, die ähnlich aufgebaut sein kann wie die Kopfbrause 5 an der Oberseite 4.

An der rechten Seitenwand 3 ist, etwas höher als an der linken Seitenwand, eine ähnliche Baueinheit 6' befestigt, bei der diesmal keine Brause vorhanden ist. Auch diese Baueinheit 6' dient dazu, Licht abzugeben, wie im folgenden noch beschrieben werden wird.

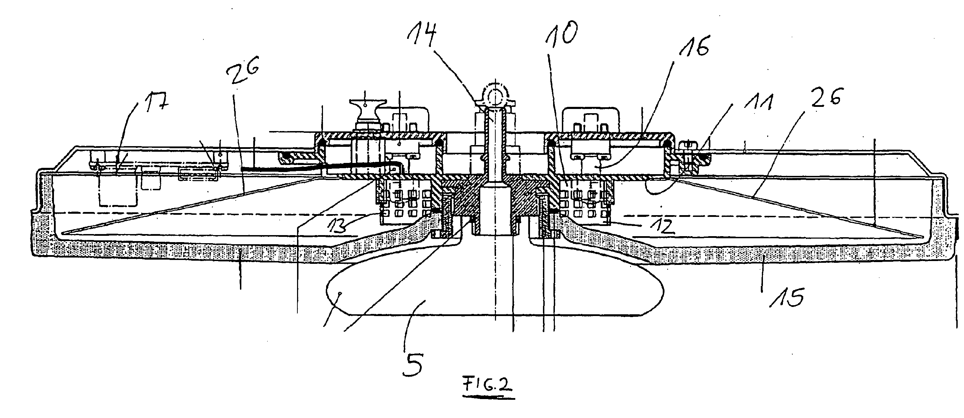

Nun zu Fig. 2. Fig. 2 zeigt in größerem Massstab eine Baueinheit 6 mit der Brause 5. Zentrales Element der Baueinheit 6 zur Abgabe von Lichtsignalen ist ein flacher hohler Quader 10 mit vier Seitenwänden, die später noch detailliert dargestellt werden. Dieser Quader 10 ist an einer Art Rückwand 11 der Baueinheit ausgebildet. Die einzelnen Seitenwände 12, die als ebene Platinen ausgebildet sind, können aus entsprechenden Halterungen entnommen werden.

Auf den durch die Platinen 12 gebildeten Seitenwänden sitzt eine Vielzahl von LED's 13, die in Reihen und Spalten angeordnet sind.

Innerhalb der Mitte des hohlen Quaders 10 ist eine Durchführung 14 zur Versorgung der Brause 5 mit Brausewasser hindurchgeführt, wobei diese Einzelheiten nicht näher dargestellt und erläutert werden.

Der Durchmesser des Brausekörpers der Brause 5 ist auf jeden Fall grösser als die Diagonale des Quaders 10, so dass von unten her, wenn man die Brause an der Oberseite 4 der Duschkabine der Fig. 1 betrachtet, der Blick auf den Quader 10 durch die Brause 5 verhindert wird.

Parallel zu der bereits erwähnten Rückwand 11 der Baueinheit 6 wird die Baueinheit nach unten hin durch eine Strahlaustrittsscheibe 15 abgeschlossen. Bei der Strahlaustrittsscheibe 15 handelt es sich um ein Element aus einem lichtdurchlässigen Material, das aber nicht durchsichtig ist.

Radial ausserhalb des Quaders 10 ist ein Reflektor 26 angeordnet, der die Form eines flachen Kegels aufweist. Der Winkel dieses Reflektors 26 ist so gewählt, dass die senkrecht aus der Fläche der Platinen 12 austretenden Strahlen durch den Reflektor so umgelenkt werden, dass dass sie auch senkrecht durch die Strahlaustrittsscheibe 15 hindurchtreten.

In radialer Richtung ausserhalb des Quaders 10 enthält die Baueinheit 6 noch insgesamt vier Lichtquellen 16 in Form von Niederspannungslampen, also Lampen, die weisses Licht geben.

In der Baueinheit ist noch eine Steuerungselektronik 17 untergebracht, die zur Ansteuerung der LED's 13 dient.

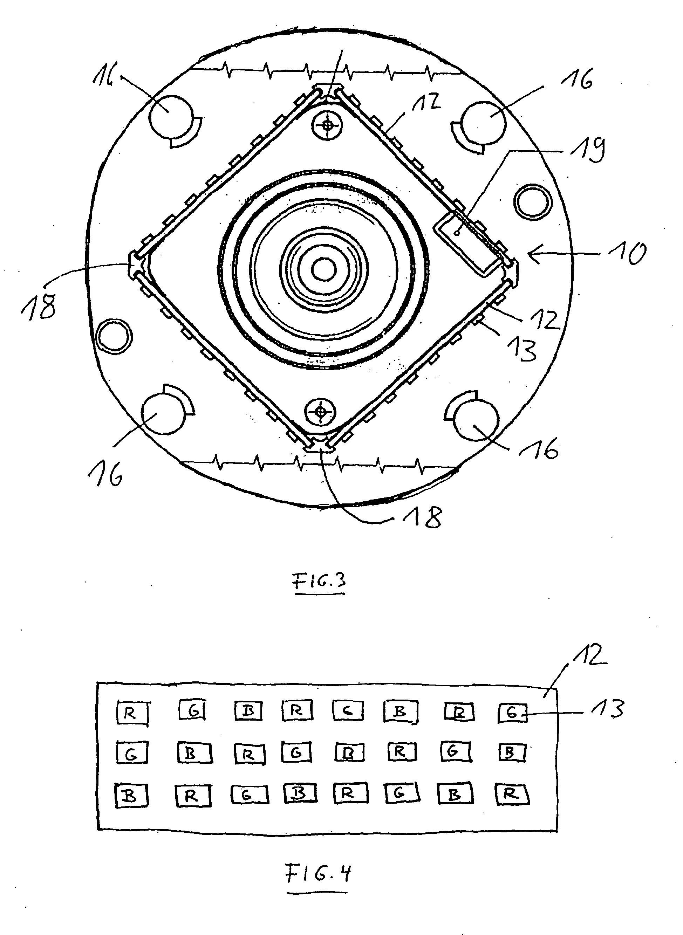

Fig. 3 zeigt in größerem Massstab eine Draufsicht auf den mittleren Bereich der Baueinheit 6. Der Quader 10 enthält vier Eckpfosten 18, die jeweils zwei unter einen Winkel von 90° verlaufende senkrecht zur Zeichnungsebene gerichtete Nuten enthalten. In diese Nuten sind die Platinen 12 eingeschoben. An der Aussenseite der Platinen 12 sind die erwähnten Lichtquellen 13 angebracht, die als LED's ausgebildet sind. Sie weisen einen geringen seitlichen Abstand voneinander auf.

Im dargestellten Beispiel wird ein Quader 10 mit vier Seitenwänden verwendet, die als ebene Platinen 12 ausgebildet sind. Es könnte auch ein flacher Zylinder statt des Quaders 10 verwendet werden, bei dem die Seitenwände dann gekrümmt verlaufen. Auch ein Quader mit einer grösseren Zahl von Seitenwänden könnte verwendet werden, um sich der Kreisform stärker anzunähern.

Die Spannungsversorgung der LED's 13 geschieht über einen Anschlussblock 19.

Fig. 4 zeigt jetzt eine Frontansicht einer Platine 12 mit den LED's. Die LED's 13 sind in drei parallelen geraden Reihen angeordnet, wobei in der obersten Reihe der Fig. 4 das Farbmuster rot, gelb, blau sich regelmäßig wiederholt.

Die mittlere Reihe ist so ausgerichtet, dass die einzelnen LED's der mittleren Reihe neben den LED's der ersten Reihe angeordnet sind. Bei der dritten Reihe ist dies genauso ausgeführt. Dadurch bilden sich auch Spalten, die senkrecht zu der Richtung der Reihen verlaufen.

In der mittleren Reihe wiederholt sich das Farbmuster der oberen Reihe, ist jedoch um ein Raster versetzt.

In der unteren Reihe wiederholt sich ebenfalls das gleiche Muster, jedoch ist hier wiederum das Muster gegenüber der unmittelbar benachbarten Reihe um ein Raster versetzt. Dadurch entsteht auch eine gleichmässige Wiederholung eines bestimmten Farbmusters in den Spalten der Anordnung von LED's.

Alle Platinen 12 können identisch ausgebildet sein, da sich das Muster nicht um die Ecken des Quaders 10 herum fortsetzen muß.

Aufgrund der engen Anordnung der Lichtquellen jeweils einer Farbe und des regelmässig sich wiederholenden Farbmusters entsteht eine echte Farbmischung, die zusammen mit der Lichtaustrittsscheibe in Form eines Diffusers dazu führt, dass der Benutzer der Einrichtung tatsächlich eine homogene Farbe sieht. Diese Farbe wird durch das Verhältnis der Ansteuerung der einzelnen LED's bestimmt. Im einfachsten Fall ist die Zahl der LED's jeder Farbe gleich. Es ist aber auch denkbar, wenn die Zahl der LED's der verschiedenen Farben unterschiedlich ist, dies durch entsprechende Ansteuerung auszugleichen.

Im dargestellten Fall werden LED's der Farben rot, grün und blau verwendet, was durch die Anfangsbuchstaben der Farben in Fig. 4 angedeutet wird.

In der Duschkabine der Fig. 1 kann ein Benutzer sich mit Kopfbrause und Seitenbrause duschen und sich dabei einer Chromotherapie unterziehen. Welche Farberscheinungen auftreten, wird durch die Steuerungselelektronik 17 bestimmt. Diese kann alle möglichen Arten von Lichtsignalen erzeugen, da alle LED's einzeln angesteuert werden können.

## Patentansprüche

1. Lichtabgabeeinrichtung, insbesondere für Duschkabinen oder dergleichen, mit
einer Vielzahl von Lichtquellen, die derart angeordnet sind, dass ein direktes Sehen der Lichtquellen (13) verhindert ist, sowie mit
einer vorzugsweise mittig angeordneten Sanitärbrause (5),
**dadurch gekennzeichnet, dass**
die Lichtquellen jeweils einfarbiges Licht abgeben,
Lichtquellen für unterschiedliche Farben vorhanden sind,
die Lichtquellen mit geringem seitlichen Abstand und
in einem sich regelmäßig wiederholenden Muster der Farben angeordnet sind.

2. Lichtabgabeeinrichtung nach Anspruch 1, bei der die Lichtquellen (13) in mindestens einer Fläche angeordnet sind, vorzugsweise einer ebenen Fläche.

3. Lichtabgabeeinrichtung nach Anspruch 1 oder 2, mit einer Lichtabgaberichtung, die quer zu der Fläche der Lichtquellen (13) verläuft.

4. Lichtabgabeeinrichtung nach einem der vorhergehenden Ansprüche, mit einer Strahlaustrittsscheibe (15), die insbesondere mindestens angenähert eben ausgebildet ist.

5. Lichtabgabeeinrichtung nach einem der vorhergehenden Ansprüche, bei der Lichtquellen (13) in mindestens einer Reihe angeordnet sind.

6. Lichtabgabeeinrichtung nach einem der vorhergehenden Ansprüche, bei der die Lichtquellen (13) in mehreren parallelen Reihen angeordnet sind, wobei das Farbmuster zwischen zwei benachbarten Reihen um ein Raster versetzt ist.

7. Lichtabgabeeinrichtung nach einem der vorhergehenden Ansprüche, bei der die Lichtquellen (13) an den Umfangsflächen eines Zylinders und/oder Quaders (10) angeordnet sind.

8. Lichtabgabeeinrichtung nach einem der vorhergehenden Ansprüche, bei der die Sanitärbrause (5) die Lichtquellen (13) abdeckt.

9. Lichtabgabeeinrichtung nach einem der vorhergehenden Ansprüche, mit einer Ansteuerungslogik (17) zur auswählbaren Ansteuerung der Vielzahl von Lichtquellen (13).

10. Lichtabgabeeinrichtung nach einem der vorhergehenden Ansprüche, mit zusätzlichen Lampen (16), insbesondere Niederspannungslampen.

## Claims

1. A light emitting device, particularly for shower cabinets or the like, with a plurality of light sources which are arranged such that direct observation of the light sources (13) is obstructed, provided with a preferably centrally located sanitary shower (5), **characterised in that** the light sources respectively emit monochromatic light, light sources are available for different colours, the light sources are arranged with small lateral distance in between and in a regularly repeated pattern of colours.

2. A light emitting device according to Claim 1, wherein the light sources (13) are arranged in at least one surface, preferably a plane surface.

3. A light emitting device according to Claim 1 or 2, with a light emitting direction that runs transversely to the surface of the light sources (13).

4. A light emitting device according to one of the preceding claims, with a spray disc (15), which in particular is at least approximately plane.

5. A light emitting device according to one of the preceding claims, wherein light sources (13) are arranged at least in one row.

6. A light emitting device according to one of the preceding claims, wherein the light sources (13) are arranged in several parallel rows, whereby the colour pattern is displaced by one grid between two neighbouring rows.

7. A light emitting device according to one of the preceding claims, wherein the light sources (13) are arranged on the circumferential surfaces of a cylinder and/or a cuboid (10).

8. A light emitting device according to one of the preceding claims, wherein the sanitary shower (5) covers the light sources (13).

9. A light emitting device according to one of the preceding claims, with activation logics (17) for selective activation of the plurality of light sources (13).

10. A light emitting device according to one of the preceding claims, with additional lamps (16), in particular low-voltage lamps.

## Revendications

1. Dispositif émetteur de lumière, notamment pour cabines de douche ou similaire, comprenant
une multitude de sources lumineuses qui sont disposées de telle manière qu'une vue directe des sources lumineuses (13) est empêchée, et comprenant
un pommeau sanitaire (5) disposé de préférence de manière centrale,
**caractérisé en ce que**
les sources lumineuses émettent chacune une lumière unicolore,
des sources lumineuses sont présentes pour différentes couleurs,
les sources lumineuses sont disposées avec un écart latéral moindre et dans un modèle des couleurs se répétant régulièrement.

2. Dispositif émetteur de lumière selon la revendication 1, dans lequel les sources lumineuses (13) sont disposées dans au moins une surface, de préférence dans une surface plane.

3. Dispositif émetteur de lumière selon la revendication 1 ou 2, ayant une direction d'émission de la lumière s'étendant transversalement par rapport à la surface des sources lumineuses (13).

4. Dispositif émetteur de lumière selon l'une quelconque des revendications précédentes, comprenant un disque de sortie de rayons (15) qui est exécuté notamment au moins approximativement de manière plane.

5. Dispositif émetteur de lumière selon l'une quelconque des revendications précédentes, dans lequel les sources lumineuses (13) sont disposées en au moins une rangée.

6. Dispositif émetteur de lumière selon l'une quelconque des revendications précédentes, dans lequel les sources lumineuses (13) sont disposées en plusieurs rangées parallèles, le modèle des couleurs entre deux rangées voisines étant décalé d'une trame.

7. Dispositif émetteur de lumière selon l'une quelconque des revendications précédentes, dans lequel les sources lumineuses (13) sont disposées sur les surfaces périphériques d'un cylindre et/ou d'un parallélépipède (10).

8. Dispositif émetteur de lumière selon l'une quelconque des revendications précédentes, dans lequel le pommeau sanitaire (5) recouvre les sources lumineuses (13).

9. Dispositif émetteur de lumière selon l'une quelconque des revendications précédentes, comprenant une logique de commande (17) pour la commande sélectionnable de la multitude de sources lumineuses (13).

10. Dispositif émetteur de lumière selon l'une quelconque des revendications précédentes, comprenant des lampes supplémentaires (16), notamment des lampes à basse tension.
